Europäisches Patentamt

(19) European Patent Office

Office européen des brevets

(11) EP 0 871 881 B1

(12) EUROPEAN PATENT SPECIFICATION

(45) Date of publication and mention
of the grant of the patent:
27.02.2002  Bulletin 2002/09

(51) Int Cl.⁷: $G01N\ 33/53$, $G01N\ 33/68$, $G01N\ 33/573$ // $C12Q1/48$

(21) Application number: 95939377.8

(22) Date of filing: 08.12.1995

(86) International application number:
PCT/IE95/00061

(87) International publication number:
WO 97/22003 (19.06.1997 Gazette 1997/26)

(54) **METHOD OF DETERMINING OR DETECTING DONOR ORGAN DAMAGE FOLLOWING XENOTRANSPLANTATION BASED ON DONOR ORGAN-DERIVED ANALYTES**

VERFAHREN ZUR BESTIMMUNG UND ZUM NACHWEIS VON SPENDERORGANSCHÄDEN NACH XENOTRANSPLANTATION AUF DER GRUNDLAGE VON AUS DEM SPENDERORGAN FREIGESETZTEN ANALYTEN

PROCEDE POUR DETERMINER OU DETECTER DES DOMMAGES A L'ORGANE DONNEUR A LA SUITE D'UNE XENOTRANSPLANTATION, A L'AIDE DES ANALYTES DERIVES DE L'ORGANE DONNEUR

(84) Designated Contracting States:
**AT BE CH DE DK ES FR GB GR IE IT LI LU MC NL PT SE**

(43) Date of publication of application:
**21.10.1998  Bulletin 1998/43**

(73) Proprietor: **Biotrin Intellectual Properties Limited Mount Merrion, County Dublin (IE)**

(72) Inventors:
  • **DOYLE, John Martin
    Deansgrange, County Dublin (IE)**
  • **KILTY, Cormac Gerard
    Sandycove, County Dublin (IE)**

(74) Representative: **Ryan, Anne Mary et al
c/o Anne Ryan & Co. 60 Northumberland Road
Ballsbridge Dublin 4 (IE)**

(56) References cited:
  **EP-A- 0 692 716**          **WO-A-93/22452**
  **WO-A-96/31779**

  • **CELL BIOLOGY AND TOXICOLOGY, vol. 10, no. 5/6, December 1994, pages 407-414, XP000578780 A.E.M. VICKERS.: "Use of human organ slices to evaluate the biotransformation and drug-induced side-effects of pharmaceuticals."**
  • **NEPHRON, vol. 67, no. 3, July 1994, pages 308-316, XP000578885 A.G.M. SUNDBERG ET AL.: "Urinary pi-class glutathione transferase as an indicator of tubular damage in the human kidney." cited in the application**
  • **TRANSPLANTATION, vol. 61, no. 6, 27 March 1996, pages 904-908, XP000578779 P. TIAINEN ET AL.: "Hepatocellular integrity in liver donors and recipiens indicated by glutathione transferase alpha."**

## Description

Technical Field

This invention relates to a method of determining or detecting the presence of a donor organ-derived analyte in a biological fluid indicative of donor organ damage following xenotransplantation.

Background Art

Currently there exists a worldwide shortage of donor organs for allotransplantation (e.g., liver, kidney and heart) and it has been proposed and demonstrated that this problem can be overcome albeit temporarily by the use of animal organs prior to orthotopic transplant surgery. Chari, R.S., *et al.* (The New England Journal of Medicine (1994); Vol. 33, No. 4 pp 234-237) treated hepatic failure with *ex vivo* pig-liver perfusion followed by orthotopic liver transplantation. One patient was stabilised for 10 days and subsequently underwent successful orthotopic liver transplantation. However, previous attempts at inter-species transplantation of organs (xenotransplantation) have not been successful because of the phenomenon known as *hyperacute rejection* whereby the newly transplanted organ is overwhelmed and rejected by the host immune system within hours of implantation into the recipient. Recent advances in the area of molecular genetics have allowed the development of transgenic animals (mainly pigs) whose organs are genetically engineered to be less immunogenic when transferred into different species (i.e., human/non-human primate) (Transplant News (1995); Vol 5, No. 9). In this report, it is postulated that immunotolerance is achieved by the expression of a human 'decay accelerating factor' gene in the transgenic organ which acts to prevent host activation of complement components (e.g.Cb3 complex) and therefore minimise hyperacute rejection. However, as with intra-species organ transplantation (allotransplantation), xenograft rejection is still an issue with which the transplant physician must grapple and is further complicated by the obvious fact that tissues from different species are present in the graft recipient.

Conventional liver function tests are incapable of differentiating between donor and recipient (e.g., porcine and human) organ-derived analytes. For example, serum alanine aminotransferase (ALT)/aspartate aminotransferase (AST) measurement does not distinguish between the porcine and human enzymes thus rendering futile any attempt to identify the enzyme source post-transplant. A similar situation would pertain in the case of renal xenotransplant analysis. In fact there is currently no generally accepted protein marker of kidney integrity.

Glutathione S-transferases (GSTs) comprise a multigene family of proteins consisting mainly of alpha ($\alpha$GST), mu ($\mu$GST), pi ($\pi$GST) and theta-class ($\theta$GST) isoforms as defined by isoelectric point and are responsible for the detoxification of a range of xenobiotics, mainly *via* conjugation to glutathione (Beckett, G.J and Hayes, J.D. Advances in Clinical Chemistry (1993); *30*, 281-380). In humans, the uniform hepatic distribution of $\alpha$GST together with relatively high hepatic levels and a short plasma half-life (approximately 1hr) means that this enzyme is more sensitive than the aminotransferases as an indicator of hepatic status following transplantation and drug-induced liver damage.

EP-A 0 640 145 discloses a method which assists in the early diagnosis of rejection in a liver transplant recipient and which comprises measuring an increase in plasma or serum $\alpha$GST from the recipient in the absence of or preceding any change in plasma or serum transaminase.

Pi Glutathione S-transferase is located in the cytoplasm of bile duct epithelial cells within the liver and is thought to exist only in the homodimeric form. This heterogenous intrahepatic GST distribution suggests that the different isoenzymes have unique *in vivo* functions in different hepatic regions and it can therefore be concluded that measurement of plasma or biliary GST levels will facilitate the monitoring of the hepatic status of an individual. A similar unique distribution also exists with respect to renal tissues where $\alpha$GST is located in the proximal tubule region and $\pi$GST is confined to the distal tubule region of the nephron (Campbell, J.A.H. *et. al.*, Cancer (Philadelphia) (1991); *67,* 1608-1613). In a recent report it was proposed that simultaneous detection of $\alpha/\pi$GST in human urine could be used to distinguish between cyclosporin A nephrotoxicity and graft rejection respectively, due to the site-specific nature of the respective tissue insult (Sundberg, A.G.M. *et al.*, Nephron, (1994): *67,* 308-316).

Vickers, A.E.M (Cell Biology and Toxicology (1994); *10:* 407-414) describes how the biotransformation of the immunosuppressant cyclosporin A was used to evaluate the metabolic competence of human liver and kidney organ slices in combination with a number of markers of cell viability and function. $\alpha$GST was used a clinical marker for liver damage. It is postulated that the monitoring of cyclosporim blood levels or the proteins metabolising the drug after transplantation could give some insight as to the quality of the liver transplanted.

WO 96/31779 describes a method for the rapid assessment of organ status, including organ damage following immunological or toxicological insult, which is based on the detection of one or more isoenzymes of GST in diverse biological fluids. The method involves contacting a particle-labelled anti-GST antibody specific for the isoenzyme and a sample of a biological fluid suspected of containing the isoenzyme, the antibody having a sensitivity sufficient to detect at least a picomolar amount of the isoenzyme and capturing the particle-labelled antibody-isoenzyme complex on an immobilised capture antibody to generate a visually detectable signal.

Accordingly, there is a need for a method of discriminating between host and graft-derived analytes as a means for detecting graft organ damage or, indeed, to distinguish between host and/or graft organ damage.

Disclosure of Invention

The invention provides a method of determining or detecting the presence of a donor organ-derived analyte in a substantially non-donor biological fluid, in the presence or absence of a corresponding recipient analyte, indicative of donor organ damage following xenotransplantation and, thereby, enabling identification of xenograft organ damage in said recipient, which method comprises capturing or detecting said donor analyte by an antibody which is:

a) specific for the donor organ-derived analyte and does not exhibit cross-reactivity with the corresponding homologous recipient analyte; or

b) capable of cross-reacting with said donor organ-derived analyte, wherein the reactivities with the donor organ-derived analyte and the homologous recipient analyte can be quantitatively distinguished.

and directly or indirectly determining the donor organ-derived analyte.

Accordingly, the method according to the invention enables one to distinguish between host-derived and donor-derived analytes and, thereby, to obtain an indication of xenotransplant status and possibly rejection, so that corrective therapy can be undertaken as appropriate as soon as such a donor organ-derived analyte is identified in a biological fluid of the recipient as will be demonstrated in greater detail hereinbelow.

By biological fluid herein is meant for example body fluids such as bile, plasma, serum and urine as well as tissue support media and perfusates. The biological fluids herein are also referred to generally as matrices.

By donor organ-derived herein is meant organs *per se* and tissue and cells which are constituent parts thereof or derived therefrom.

Likewise by donor material herein is meant organs *per se* and tissue and cells which are constituent parts thereof or derived therefrom.

Thus, by way of example, the donor material can be a liver, a part of a liver such as a lobe or hepatocytes. Typically in the case of hepatocytes, an hepatocyte cartridge would be used.

Generally, the recipient will be a human being or a non-human primate.

Accordingly, the donor material will preferably be derived from a non-human primate or an animal such as the pig which is physiologically and biochemically similar to the human being.

The donor animal can be a transgenic animal.

The xenotransplantation can be *in vivo* or *ex vivo*

The organ which is xenotransplanted is suitably a heart, kidney or liver.

Preferably, the donor organ-derived analyte is a protein, more especially an enzyme.

Preferably, the donor organ-derived analyte will be donor specific. In the event that there is a corresponding recipient analyte, preferably the recipient analyte has a low degree of homology with said donor organ-derived analyte. However, the donor organ-derived analyte can have a high degree of homology (for example, greater than 80% homology) with the corresponding recipient analyte and yet is detectable in accordance with the invention as hereinafter described.

A preferred enzyme is a glutathione S-transferase, more especially αGST.

Further, preferably, determination of the captured donor organ-derived analyte is carried out by an immunoassay format. Suitable immunoassay formats include enzyme immunoassays.

Thus, in the case of porcine αGST in a human biological fluid, the porcine αGST can be captured by IgG [anti-porcine αGST] directly or indirectly bound to a solid phase or, alternatively, by IgG [anti-human αGST] exhibiting cross-reactivity.

We have raised rabbit polyclonal IgG [anti-porcine αGST] which is highly specific for porcine αGST. In the event that the antibody also displays a small degree (~5-10%) cross-reactivity with human αGST, such as in the presence of a high concentration of human αGST, then the human αGST can be separately determined and the amount of porcine αGST correctly calculated as hereinafter described.

We have identified a monoclonal anti-human αGST with almost 100% cross-reactivity with porcine αGST as hereinafter described in Example 3.

The enzyme immunoassay can be a sandwich format or a semi-competitive enzyme immunoassay as hereinafter described in Examples 1 and 2, respectively.

In a further aspect of the invention, a donor material is tested for viability prior to xenotransplantation.

Thus, the invention also enables one to test the viability of donor material by measuring a donor organ-derived analyte in a biological fluid which perfuses the donor material by adopting a method as hereinbefore defined.

In this aspect of the invention one can detect an analyte in accordance with any known method in addition to the methods according to the invention. For example, if the organ-derived analyte is an enzyme, then an enzyme assay based on the use of a substrate for the enzyme can be used to detect said organ-derived analyte.

Brief Description of Drawings

In the accompanying drawings:

Fig. 1 is a schematic diagram of the sandwich enzyme immunoassay of Example 1;

Fig. 2 is a plot of absorbance at 450/630 nm *versus* αGST concentration (μg/l) according to the sandwich immunometric enzyme immunoassay for porcine αGST of Example 1;

Fig. 3 is a schematic diagram of the semi-competitive enzyme immunoassay of Example 2;

Fig. 4 is a plot of absorbance at 450/630 nm *versus* αGST concentration (μg/l) according to the competitive immunometric enzyme immunoassay for porcine αGST of Example 2;

Fig. 5 is an SDS-PAGE analysis of human and porcine αGST;

Fig. 6 is an immunoblot analysis of human and porcine αGST using IgG [anti-human αGST] and goat IgG [anti-rabbit IgG]-HRP conjugate;

Fig. 7 is an immunoblot analysis of human and porcine αGST using IgG [anti-porcine αGST] and goat IgG [anti-rabbit IgG]-HRP conjugate; and

Fig. 8 is an immunoblot analysis of human and porcine αGST using IgG [anti-human αGST] and goat IgG [anti-mouse IgG]-HRP conjugate.

Modes for Carrying Out the Invention

The invention will be further illustrated by the following Examples.

Preparatory Example A

Purification of porcine αGST

Alpha GST was purified from porcine liver by affinity chromatography and chromatofocussing (pH 9.5 - 6.0). Precise details of the purification procedure are as follows:

a. 30g of porcine liver was homogenised for 2 minutes in homogenisation buffer, at a ratio of one part liver to three parts buffer, using a Waring (Waring is a Trade Mark) blender. The homogenisation buffer had the following composition:

10mM Tris-HCl
250mM Sucrose
5mM EDTA        pH 7.8
2μg/ml Leupeptin
2μg/ml Pepstatin.

b. The liver homogenate was centrifuged at 10000g for 60 minutes.

c. The supernatant was then loaded on a Glutathione(GSH)-Sepharose Affinity column previously equilibrated in 10mM Tris pH9.1. Equilibration buffer was reapplied to elute unbound protein. Finally 50mM Tris pH9.1 containing 5mM GSH was used to elute bound GST from the affinity column.

d. The eluted material was then dialysed against 25mM ethanolamine pH9.5 and applied to a chromatofocussing column (PBE94 supplied by Pharmacia). Elution buffer was Polybuffer 96 (Polybuffer 96 is a Trade Mark) prepared according to manufacturer's instructions.

Preparatory Example B

Antibody Production and Purification:

Purified porcine αGST was injected into New Zealand White rabbits subcutaneously (s.c.) according to the time schedule given below and serum evaluated for anti-αGST reactivity. Once the IgG [anti-porcine αGST] titre was sufficient as determined by semi-quantitative dot blot analysis, the animals were exsanguinated and serum collected. Total IgG was purified from rabbit serum by Protein A affinity chromatography and was used for both microtitre plate coating (semi-competitive EIA - Example 2) and biotinylation (sandwich EIA - Example 1). Monoclonal IgG [anti-human αGST], as ascites, was obtained from The University Hospital, Nijmegen, The Netherlands and was not purified further prior to use.

Immunisation Schedule (general):

*Day 1:* A test bleed of 5ml preserum from the ear of the rabbit was carried out and then 0.5ml of porcine αGST antigen (100μg) was mixed with an equal volume of Freund's Complete Adjuvant. The antigen and adjuvant were homogenised to ensure good emulsion. This mixture was then injected s.c. into multiple sites on the back of the rabbit which had previously been shaved.

*Day 28:* A test bleed of 5ml serum from the ear of the rabbit was carried out and then 0.5ml antigen (100μg) was mixed with an equal volume of Freund's Complete Adjuvant. The antigen and adjuvant were homogenised to ensure good emulsion. This mixture was then injected s.c. into multiple sites on the back of the rabbit.

*Day 42:* A test bleed of 10ml blood was taken from the rabbit's ear.

*Day 56:* A second boost was given to the rabbit as described on Day 28.

*Day 70:* A test bleed of 10ml of blood was taken from the ear of the rabbit. When the titre was sufficiently high, the rabbit was sacrificed and as much blood as possible was collected

Preparatory Example C

Immunoblotting

All polyclonal and monoclonal IgGs for use in the following Examples were checked for reactivity and cross - reactivity against both human and porcine αGST, respectively *via* the following immunoblot combinations-

(a) Rabbit IgG [anti-human αGST] was used to probe nitrocellulose membranes containing immobilised human and porcine αGST.

(b) Rabbit IgG [anti-porcine αGST] was used to probe nitrocellulose membranes containing immobilised human and porcine αGST.

(c) Murine IgG [anti-human αGST] was used to probe nitrocellulose membranes containing immobilised human and porcine αGST.

The method used for Immunoblot detection was as follows:

1. Both human and porcine αGST (0.5μg/track) were electrophoresed on 15% SDS-PAGE and molecular weight markers were included.

2. After electrophoresis, the polyacrylamide gel was cut and one half stained for protein while the remainder was used for electrophoretic transfer onto nitrocellulose.

3. After electrophoretic transfer, the nitrocellulose membranes were blocked for 1 hour with 5%(w/v) Marvel (Marvel is a Trade Mark) in phosphate buffered saline containing 0.05%(w/v) TWEEN-20® (PBST)- blocking buffer.

4. The following solutions were then prepared:

(i) Rabbit IgG [anti-human $\alpha$GST] in 1%(w/v) Marvel in PBST
(ii) Rabbit IgG [anti-porcine $\alpha$GST] in 1%(w/v) Marvel in PBST
(iii) Murine IgG [anti-human $\alpha$GST] in 1%(w/v) Marvel in PBST

and added to the membranes, once blocking buffer was decanted. Incubation with antibody solutions was allowed to proceed for one hour.

5. The nitrocellulose membranes were then washed in PBST (2x for 5 min. each).

6. Anti-rabbit IgG - HRP conjugate was then prepared (1/1000 in 1% (w/v) Marvel in PBST and added to 4(i) and (ii) above. Anti-murine IgG -HRP conjugate was also prepared (1/1000) and added to 4(iii) above. The anti-species (rabbit/mouse) conjugates used for the immunodetection were obtained from Bio-Rad Laboratories Limited.

7. After one hour incubation with anti - species conjugates, the reagents were discarded and the membranes washed as in 5 above.

8. Diaminobenzidine substrate was then prepared and added to the membrane. A positive reaction was indicated by a brown precipitate on the nitrocellulose membrane.

Preparatory Example D

Porcine $\alpha$GST-horseradish peroxidase (HRP) conjugate synthesis:

$\alpha$GST-HRP conjugates were synthesised using thioether conjugation methodology. Reactive maleimide groups were introduced onto $\alpha$GST molecules using SMCC (succinimidyl 4-(N-maleimidomethyl) cyclohexane 1-carboxylate) and masked sulphydryl groups were linked to HRP (Duncan, R.J.S., *et al* (1983); Anal. Biochem. *132*, 68-73). After a demasking step to produce reactive sulphydryl groups, the maleimide-activated $\alpha$GST and HRP-SH were mixed together and allowed to react for 4.5 hours. The resultant $\alpha$GST-HRP conjugate, formed by covalent thioether linkage, was brought to 50% (v/v) glycerol and stored at -20°C for use in the semi-competitive EIA of Example 2.

Preparatory Example E

Biotinylation of IgG [anti-porcine $\alpha$GST]

Biotinylated IgG [anti-porcine $\alpha$GST] was prepared by coupling purified polyclonal IgG[anti-porcine $\alpha$GST], previously generated by immunisation of rabbits with purified $\alpha$GST (as described in Preparatory Example B), to N-hydroxysuccinimide-Biotin (NHS-Biotin) under alkaline conditions. Unreacted NHS-Biotin was then removed by extensive dialysis and biotinylated IgG aliquotted and stored frozen at -20°C until required.

Example 1

Sandwich enzyme immunoassay

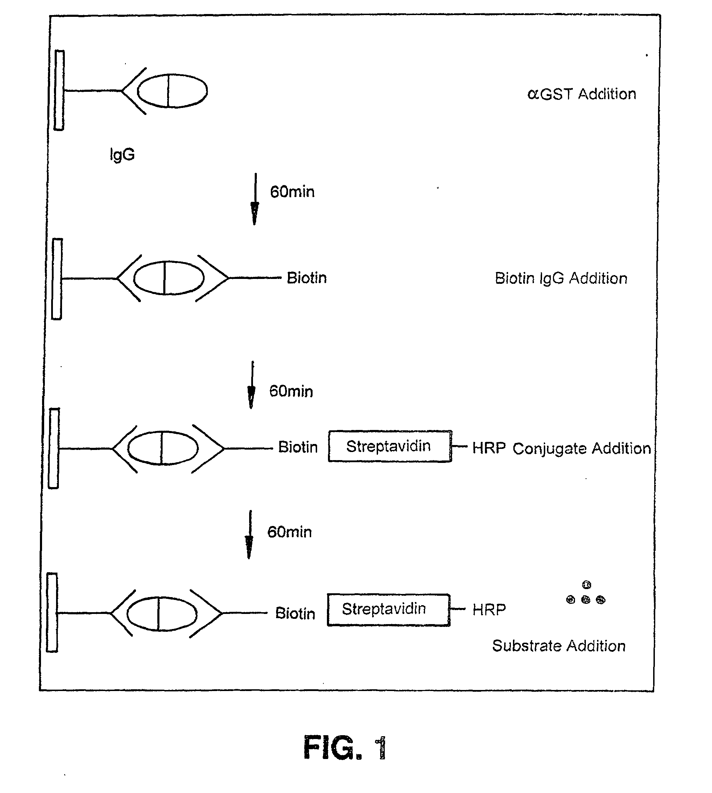

The procedure used is depicted schematically in Fig. 1.

a. A Nunc Maxisorp (Nunc Maxisorp is a Trade Mark) microtitre plate was coated with murine monoclonal IgG [anti-human $\alpha$GST] (referred to in Preparatory Example B) immobilised *via* goat F(ab)$_2$ fragments [anti-mouse IgG]. This method of antibody coating serves to orientate Mab binding sites and also improves assay sensitivity by minimising adherence - induced denaturation of the capture antibody. Microtitre plates were blocked with a protein/sucrose solution.

b. Porcine $\alpha$GST, purified from liver as described in Preparatory Example A obtained immediately after expiration and stored at 2-8°C or -20°C, was used as the assay calibrator.

c. Biotinylated IgG[anti-porcine $\alpha$GST] conjugates were used to facilitate detection of captured/immobilised $\alpha$GST.

d. A Streptavidin - horseradish peroxidase (HRP) conjugate was then used to detect the entire antigen sandwich complex in association with the use of tetramethylbenzidine substrate (TMB).

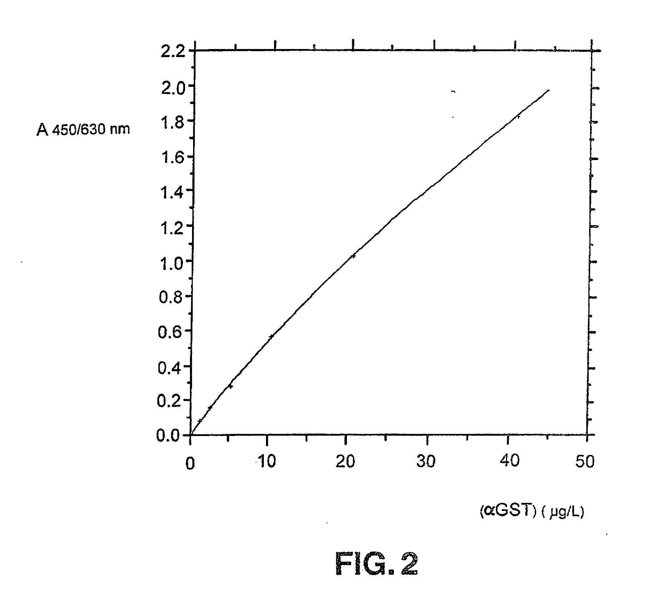

e. The enzyme reaction was stopped by the addition of 1N $H_2SO_4$ and the absorbance measured at 450nm using 630nm as a reference wavelength. Colour intensity is proportional to $\alpha$GST concentration and after generating a plot of $A_{450/630nm}$ *versus* concentration (µg/l), the concentration of unknown samples can be determined (see Fig. 2). Total assay time was less than 3.5 hours.

Example 2

Semi-Competitive Enzyme Immunoassay

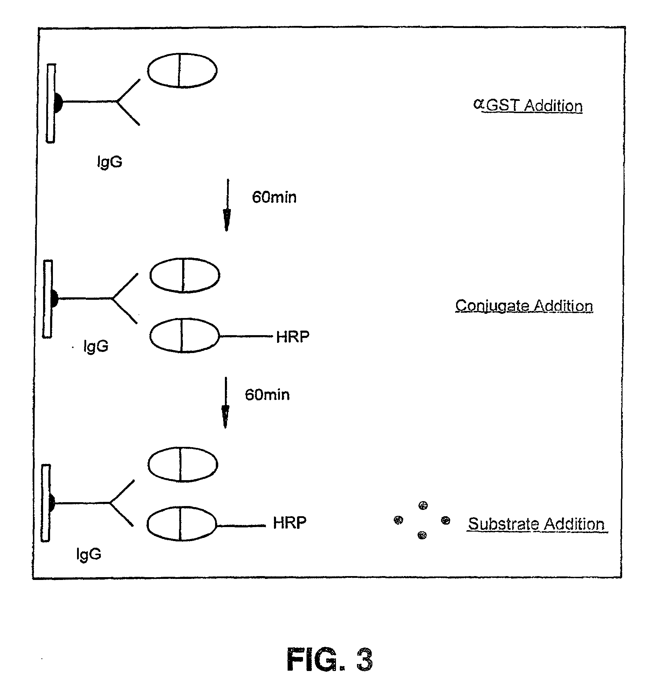

The procedure used is depicted schematically in Fig. 3.

a. In this case, Nunc Maxisorp plates were coated with polyclonal IgG [anti-porcine $\alpha$GST] immobilised *via* protein A since it was found that direct coating of polyclonal IgG did not facilitate $\alpha$GST capture, possibly due to IgG denaturation upon binding to the microtitre plate.

b. $\alpha$GST, labelled with HRP was used as the conjugate in this case and was added to the microwell subsequently to the unlabelled calibrator/sample. In this way a competition-type reaction was set up between the HRP-labelled and unlabelled $\alpha$GST.

c. After a suitable incubation period, normally 60 minutes, the microtitre plate was washed and TMB substrate was added.

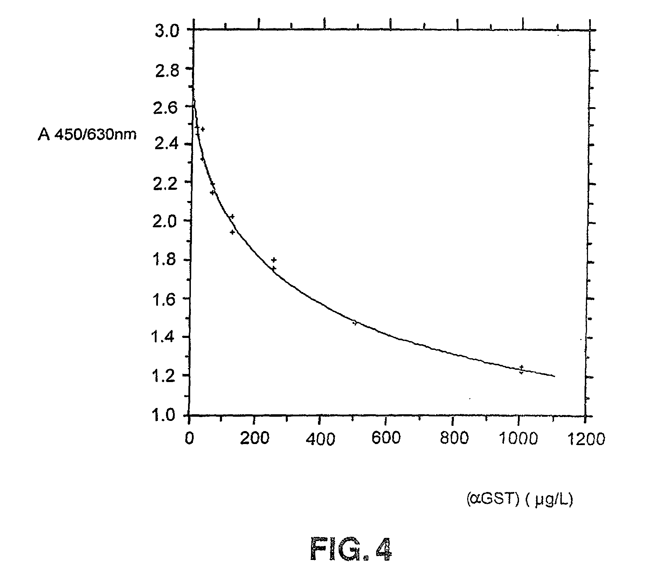

d. The enzyme reaction was stopped by the addition of 1N $H_2SO_4$ and the absorbance measured at 450nm using 630nm as a reference wavelength. Colour intensity is inversely proportional to porcine $\alpha$GST concentration and after plotting $A_{450/630nm}$ *versus* concentration (µg/l), quantitation of unknown samples is achieved (see Fig. 4).

Example 3

Assessment of purity of immunoassay reagents

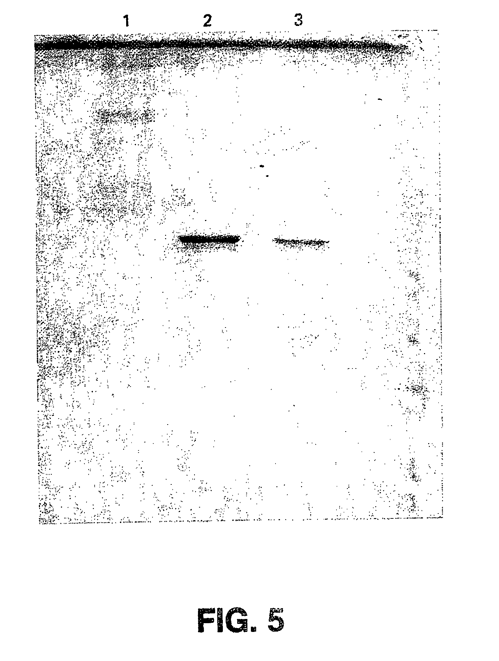

Immunoblotting was carried out in accordance with the procedure described in Preparatory Example C. The results are depicted in Figs. 5-8.

Key to tracks in Figs. 5-8:

Track 1: Molecular weight markers.
Track 2: Human $\alpha$GST (0.5µg).
Track 3: Porcine $\alpha$GST (0.5µg).

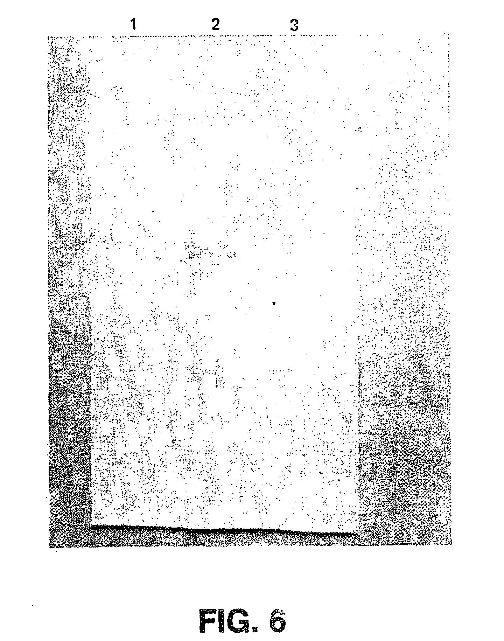

Fig. 5 illustrates the purity (indicated by the single band in each case) of both human and porcine $\alpha$GST prior to immunisation into rabbits and confirms the absence of any other human or porcine-derived proteins which might otherwise contribute to reduced assay specificity. Immunoblot analysis of the antibody reactivity reveals that the IgG [anti-human $\alpha$GST] is highly specific for human $\alpha$GST and does not exhibit any significant cross-reactivity with porcine $\alpha$GST (Fig. 6), as indicated by the strong intensity of the human $\alpha$GST signal relative to the weak intensity of the porcine $\alpha$GST signal. A finding supported by the lack of porcine $\alpha$GST ($\alpha$GST)p reactivity in a human $\alpha$GST ($\alpha$GST) h-specific enzyme immunoassay marketed by Biotrin International Limited - Mount Merrion, County Dublin, Ireland, under the name HEPKIT. The results are shown in Table 1 which represent an evaluation of $\alpha$GSTp reactivity in the Biotrin HEPKIT. It is clear that no cross-reactivity occurs when $\alpha$GSTp is assayed in the HEPKIT.

Table 1

| [$\alpha$GST]h (µg/l) | $A_{450/630nm}$ |
|---|---|
| 0.00 | 0.069 |
| 1.25 | 0.156 |
| 2.50 | 0.332 |

Table 1   (continued)

| [$\alpha$GST]h (µg/l) | A$_{450/630nm}$ |
|---|---|
| 5.00 | 0.627 |
| 10.0 | 1.088 |
| 20.0 | 1.495 |
| 40.0 | 1.762 |
| PC | 0.839 (6.9 µg/l) |

| [$\alpha$GST]p (µg/l) | A$_{450/630nm}$ |
|---|---|
| 8 | 0.012 |
| 40 | 0.010 |
| 200 | 0.012 |
| 1000 | 0.014 |
| PC = Positive Control. | |

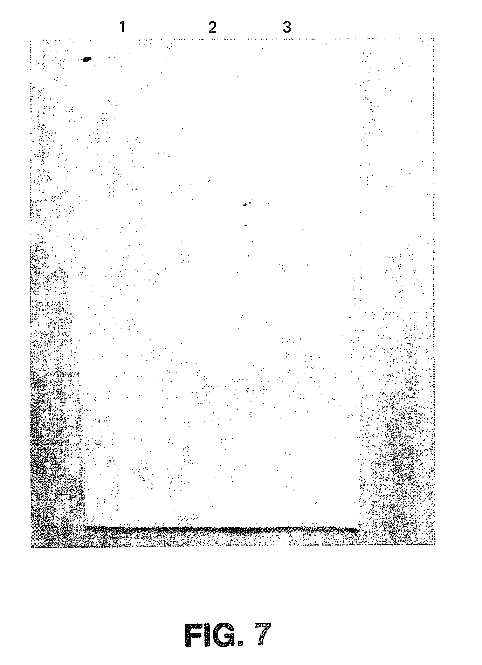

The significance of this fact is of the utmost importance since it implies that the enzyme immunoassay for human $\alpha$GST quantitation is specific for the detection of human $\alpha$GST. Thus, any human $\alpha$GST present in samples for which porcine $\alpha$GST will also be measured, can be specifically detected without cross-contamination from the porcine antigen. This lack of cross-reactivity in the human $\alpha$GST enzyme immunoassay and the finding that about 5-10% cross-reactivity is evident between IgG[anti-porcine $\alpha$GST] and human $\alpha$GST (Fig. 7), as indicated by the weak intensity of the human $\alpha$GST signal relative to the strong intensity of the porcine $\alpha$GST signal, means that simultaneous quantitation of human $\alpha$GST in the HEPKIT enzyme immunoassay and porcine $\alpha$GST in the enzyme immunoassay for porcine $\alpha$GST can be used to correct for any human $\alpha$GST contribution in the porcine $\alpha$GST immunoassay.

For example, if a sample gives the following readings:

| Assay | $\alpha$GST(µg/l) |
|---|---|
| Porcine EIA (porcine $\alpha$GST) | 10000 |
| Hepkit EIA (human $\alpha$GST) | 1000 |

then assuming approximately 7% cross-reactivity of human $\alpha$GST in porcine EIA implies that human $\alpha$GST contributes $\frac{1000}{100}$ x 7 = 70µg/l to the porcine $\alpha$GST reading. Therefore,

$$10000 - 70 = 9930 \ \mu g/l$$

porcine $\alpha$GST present

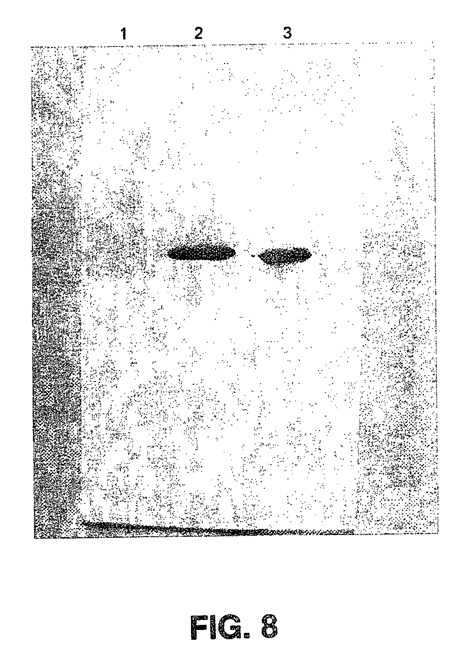

Furthermore as mentioned above, the extensive cross-reactivity between murine IgG [anti-human $\alpha$GST] and porcine $\alpha$GST is clearly illustrated in Fig. 8, as indicated by the comparable intensity of the respective bands/signals. This phenomenon is taken advantage of by using this antibody in the sandwich immunoassay of Example 1 for porcine $\alpha$GST as the 'capture' or plate-coating antibody.

Example 4

Assay specificity

As the assays of Examples 1 and 2 will primarily be used to detect porcine $\alpha$GST in non-porcine biological fluids it is essential that assay sensitivity and specificity be evaluated using porcine $\alpha$GST present in the following matrices:-

- Human urine
- Human serum
- Human plasma
- Human bile
- Tissue support media

To achieve this analysis, porcine αGST was 'spiked' into all of the above matrices and subsequently assayed in the immunoassays of Examples 1 and 2. In addition to quantitative αGST detection, assay linearity (parallelism) was also evaluated as was assay sensitivity. Furthermore, it is also necessary that assay specificity facilitates detection of porcine αGST only and that any human αGST does not cause significant interference in the immunoassay. To this end potential cross-reactivity of human αGST was investigated by adulterating porcine αGST containing samples with different amounts of human αGST to evaluate potential cross-reactivity. Given that such a significant sequence homology exists between both isoenzymes this is far from a trivial problem and can only be overcome by extensive analysis of antisera [anti-porcine αGST] for potential cross-reactivity with human αGST.

The results of the quantitation of porcine αGST in various sample matrices as determined by the sandwich and semi-competitive enzyme immunoassays are shown in Table 2.

Table 2

| RECOVERY OF SPIKED SAMPLES IN SANDWICH AND SEMI-COMPETITIVE PORCINE αGST ASSAYS: | | | | | | | | | | |
|---|---|---|---|---|---|---|---|---|---|---|
| Expected Value | Sample Diluent (µg/l) | | Plasma-human (µg/l) | | MEM (complete) (µg/l) | | TC-100 (complete) (µg/l) | | Urine-human (µg/l) | |
| (µg/l) | sand. | comp. | sand. | comp. | sand. | comp. | sand. | comp. | sand. | comp. |
| 0 | 61.4 | 19.4 | 0 | - | 0 | 46.8 | 0 | 73 | 0 | 84 |
| 4000 | 4530 | 3853 | 4299 | over | 4135 | 3643 | 4754 | 3889 | 4189.5 | 3936 |
| 2000 | 2980 | 2699 | 2500 | over | 2262 | 2420 | 2059 | 2071 | 2165.2 | 1912 |
| 1000 | - | - | 1041 | over | 1180 | 1247 | 983 | 953 | 844.5 | 857 |
| 500 | 507 | 446 | 249 | over | 512 | 485 | 540 | 562 | 392.8 | 454 |
| 250 | 279 | 260 | 85 | over | 290 | 211 | 273 | 266 | 151.9 | 218 |
| 125 | 128 | 140 | 0 | over | 136 | 188 | 197 | 163 | 17.9 | 126 |

**Key:**

1. Sand: Sandwich enzyme immunoassay.
2. Comp: Semi-competitive enzyme immunoassay.
3. Samples diluted 1/5 for Competitive Assay range (0 - 1000µg/l) then further diluted to 1/100 for Sandwich Assay (range 0-40µg/l).
4. MEM and TC-100: Tissue support media.

It is clear from Table 2 that quantitative recovery of porcine αGST is achievable in all matrices tested. Obviously it is essential that αGST is detectable in both human plasma and urine, respectively so as to facilitate xenograft liver and kidney status, and this is, indeed, the case. However, it is noticeable that the plasma matrix appears to be incompatible with the semi- competitive immunoassay format, possibly due to IgG interaction with immobilised Protein A resulting in anomalous readings. Apart from this all other fluids appear to be compatible with both assay formats. Of particular interest is the ability to detect porcine αGST in tissue support media which should facilitate the possibility of pre-transplant xenograft evaluation or isolated porcine hepatocyte viability analysis (present in cartridges), as these systems are generally maintained in conventional support media. Additionally, this matrix/sample compatibility should greatly facilitate the detection of αGST in the media used for the maintenance of *ex vivo* donor organs whereby the donor organ (e.g., liver) is maintained extra-corporeally in contact with culture medium.

Example 5

Evaluation of human αGST interference in the porcine αGST sandwich enzyme immunoassay

Evaluation of human αGST interference in the porcine αGST sandwich enzyme immunoassay of Example 1 was carried out. No assay interference, as measured by increased absorbance values, was evident at microwell concentrations less than 25µg/l (equivalent to 125µg/l sample concentration at 1/5 dilution in sample diluent). The results are shown in Table 3.

Table 3

| [αGST]p | [αGST]h | Absorbance$_{450/630nm}$ |
|---------|---------|--------------------------|
| (µg/l) | | |
| 20 | - | 0.869 |
| 20 | 6 | 0.875 |
| 20 | 12 | 0.875 |
| 20 | 25 | 0.881 |
| 20 | 50 | 0.975 |

Key:

αGSTp - porcine αGST
αGSTh - human αGST

The results shown in Table 3 demonstrate the specificity of the immunoassays for the detection of porcine αGST. It is evident from this data that human αGST does not interfere in the enzyme immunoassay even at sample concentrations as high as 125µg/l (25µg/l x 5) which represents a value of 15 and 6 times the upper limit of normal for human serum and urinary αGST, respectively. At higher levels of human αGST (>250µg/L; 50µg/l x 5) it is apparent that some interference is occurring and it has been calculated that there is about 5-10% cross-reactivity between human αGST and IgG [anti-porcine αGST]. However, co-measurement of αGST levels in quantitative immunoassays for both human and porcine αGST should allow the precise amount of human αGST present to be calculated and thus taken into account during porcine αGST quantitation in human biological fluids.
It should be further noted that the immunoassays according to the invention can also be used for the detection of porcine GST in porcine-derived biological fluids as would be the case for *in vitro* or *in vivo* toxicological studies involving pigs or pig organs. This finding is of significance since it is known that porcine and human physiology/biochemistry are quite similar and consequently pigs are often used as animal models to predict drug toxicity in humans.

**Claims**

1.  A method of determining or detecting the presence of a donor organ-derived analyte in a substantially non-donor biological fluid, in the presence or absence of a corresponding recipient analyte, indicative of donor organ damage following xenotransplantation and, thereby, enabling identification of xenograft organ damage in a recipient, which method comprises capturing or detecting said donor analyte by an antibody which ist either

    a) specific for the donor organ-derived analyte and does not exhibit cross-reactivity with the corresponding homologous recipient analyte; or

    b) capable of cross-reacting with said donor organ-derived analyte, wherein the reactivities with the donor organ derived analyte and the homologous recipient analyte can be quantitatively distinguished.

    and directly or indirectly determining the donor organ-derived analyte.

2.  A method according to Claim 1, wherein the recipient is a human being.

3.  A method according to Claim 1, wherein the recipient is a non-human primate.

4.  A method according to any one of Claims 1-3, wherein a donor organ is derived from a pig.

5.  A method according to any one of Claims 1-4, wherein the organ is a kidney.

6.  A method according to any one of Claims 1-4, wherein the organ is a liver.

7. A method according to any preceding claim, wherein the donor organ-derived analyte is a protein.

8. A method according to Claim 7, wherein the donor organ-derived analyte is an enzyme.

9. A method according to Claim 8, wherein the enzyme is a glutathione S-transferase (GST).

10. A method according to Claim 9, wherein the enzyme is an alpha GST.

11. A method according to any preceding claim, wherein the donor organ is derived from pig and wherein the antibody is polyclonal anti-porcine αGST.

12. A method according to any one of Claims 1-10 when dependent on Claim 2, wherein the antibody is anti-human αGST which exhibits substantially 100% cross-reactivity with porcine αGST.

13. A method according to Claim 11, wherein any human αGST present is detected using specific anti-human αGST in a separate assay system.

14. A method according to any preceding claim, wherein a donor material is tested for viability prior to xenotransplantation.


**Patentansprüche**

1. Verfahren zur Bestimmung oder Ermittlung der Anwesenheit eines von einem Spenderorgan stammenden Analyten in einem im Wesentlichen nicht zum Spender gehörenden biologischen Fluid in Gegenwart oder Abwesenheit eines entsprechenden Empfängeranalyten, welcher auf einen Spenderorgan-schaden nach einer Xenotransplantation hinweist und dadurch die Erkennung eines Schadens des transplantierten Fremdorgans in einem Empfänger ermöglicht, wobei das Verfahren die Aufnahme oder Ermittlung des Donoranalyten durch einen Antikörper umfasst, wobei der Antikörper entweder

a) spezifisch für den vom Spenderorgan stammenden Analyten ist und keine Kreuzreaktivität mit dem entsprechenden homologen Empfängeranalyten zeigt; oder
b) befähigt ist, mit dem vom Spenderorgan stammenden Analyten eine Kreuzreaktion einzugehen, wobei die Reaktivitäten mit dem vom Spenderorgan stammenden Analyten und dem homologen Empfängeranalyten quantitativ unterscheidbar sind,

und direkt oder indirekt den vom Spenderorgan stammenden Analyten bestimmt.

2. Verfahren nach Anspruch 1, wobei der Empfänger ein menschliches Wesen ist.

3. Verfahren nach Anspruch 1, wobei der Empfänger ein nichtmenschlicher Primat ist.

4. Verfahren nach einem der Ansprüche 1 bis 3, wobei das Spenderorgan von einem Schwein stammt.

5. Verfahren nach einem der Ansprüche 1 bis 4, wobei das Organ eine Niere ist.

6. Verfahren nach einem der Ansprüche 1 bis 4, wobei das Organ eine Leber ist.

7. Verfahren nach einem der obigen Ansprüche, wobei der vom Spenderorgan stammende Analyt ein Protein ist.

8. Verfahren nach Anspruch 7, wobei der vom Spenderorgan stammende Analyt ein Enzym ist.

9. Verfahren nach Anspruch 8, wobei das Enzym eine Glutathion-S-Transferase (GST) ist.

10. Verfahren nach Anspruch 9, wobei das Enzym eine alpha-GST ist.

11. Verfahren nach einem der vorstehenden Ansprüche, wobei das Spenderorgan von einem Schwein stammt und wobei der Antikörper eine polyklonale anti-porcin-αGST ist.

**12.** Verfahren nach einem der Ansprüche 1 bis 10, wenn abhängig von Anspruch 2, wobei der Antikörper eine anti-human-αGST ist, welche eine im wesentlichen 100%-ige Kreuzreaktivität mit porcin-αGST aufweist.

**13.** Verfahren nach Anspruch 11, wobei eine beliebige vorhandene human-αGST mittels spezifischer anti-human-αGST in einem getrennten Versuchssystem bestimmt wird.

**14.** Verfahren nach einem der vorstehenden Ansprüche, wobei ein Spendermaterial vor der Xenotransplantation einer Lebensfähigkeitsüberprüfung unterzogen wird.

**Revendications**

**1.** Méthode de détermination ou de détection de la présence d'un analyte dérivé d'un organe de donneur dans un fluide biologique essentiellement non donneur, en présence ou en l'absence d'un analyte correspondant du receveur, pouvant indiquer une lésion de l'organe de donneur après xénotransplantation, et par suite, permettant d'identifier la lésion de l'organe transplanté par xénogreffe chez un receveur, ladite méthode comprenant la capture ou la détection dudit analyte de donneur par un anticorps qui est

a) soit spécifique à l'analyte dérivé de l'organe du donneur et ne présente pas de réactivité croisée avec l'analyte homologue correspondant du receveur ;

b) soit capable d'entrer en réaction croisée avec ledit analyte dérivé de l'organe de donneur, les réactivités avec l'analyte dérivé de l'organe de donneur et l'analyte homologue du receveur pouvant être distinguées de façon quantitative,

et permettant la détermination directe ou indirecte de l'analyte dérivé de l'organe de donneur.

**2.** Méthode selon la revendication 1, dans laquelle le receveur est un être humain.

**3.** Méthode selon la revendication 1, dans laquelle le receveur est un primate non humain.

**4.** Méthode selon l'une quelconque des revendications 1 à 3, dans laquelle l'organe de donneur est d'origine porcine.

**5.** Méthode selon l'une quelconque des revendications 1 à 4, dans laquelle l'organe est un rein.

**6.** Méthode selon l'une quelconque des revendications 1 à 4, dans laquelle l'organe est un foie.

**7.** Méthode selon l'une quelconque des revendications précédentes, dans laquelle l'analyte dérivé de l'organe de donneur est une protéine.

**8.** Méthode selon la revendication 7, dans laquelle l'analyte dérivé de l'organe de donneur est une enzyme.

**9.** Méthode selon la revendication 8, dans laquelle l'enzyme est la glutathione S-transférase.

**10.** Méthode selon la revendication 9, dans laquelle l'enzyme est une alpha GST.

**11.** Méthode selon l'une quelconque des revendications précédentes, dans laquelle l'organe de donneur est d'origine porcine et l'anticorps est l'α-GST polyclonale anti-porcine.

**12.** Méthode selon l'une quelconque des revendications 1 à 10 lorsqu'elles sont dépendantes de la revendication 2, dans laquelle l'anticorps est l'α-GST anti-humaine qui présente une réactivité croisée sensiblement égale à 100 % avec l'α-GST porcine.

**13.** Méthode selon la revendication 11, dans laquelle toute α-GST humaine présente est détectée à l'aide d'α-GST spécifique anti-humaine dans un système de test séparé.

**14.** Méthode selon l'une quelconque des revendications précédentes, dans laquelle la viabilité d'un organe de donneur est testée avant xénotransplantation.

**FIG. 1**

FIG.2

**FIG. 3**

FIG.4

FIG. 5

**FIG. 6**

FIG. 7

FIG. 8